# EUROPÄISCHE PATENTSCHRIFT

(11) **EP 0 977 513 B1**
(45) Veröffentlichungstag und Bekanntmachung des Hinweises auf die Patenterteilung: **29.11.2000**
(21) Anmeldenummer: 99903668.4
(22) Anmeldetag: 22.01.1999
(51) Int. Cl.: A61B 17/28

(54) **MEDIZINISCHES SCHIEBESCHAFTINSTRUMENT**
MEDICAL SLIDING SHAFT INSTRUMENT
INSTRUMENT MEDICAL A TIGE COULISSANTE

(30) Priorität: 22.01.1998 DE 19802145
(43) Veröffentlichungstag der Anmeldung: 09.02.2000
(73) Patentinhaber: Karl Storz GmbH & Co. KG, 78532 Tuttlingen (DE)
(72) Erfinder: STIHL, Ewald, D-78187 Geisingen (DE); EFINGER, Andreas, D-78604 Rietheim (DE)
(74) Vertreter: Heuckeroth, Volker
(86) Internationale Anmeldenummer: EP9900398
(87) Internationale Veröffentlichungsnummer: WO9937221

(56) Entgegenhaltungen:
- EP-A- 0 706 780
- US-A- 5 653 713

## Beschreibung

Die Erfindung betrifft ein medizinisches Schiebeschaftinstrument, mit einem Schiebeschaft, der zwei Seite an Seite angeordnete und relativ zueinander verschiebbare Schiebelemente aufweist, mit zumindest einem Maulteil am distalen Ende des Schiebeschafts, das mittels einer Handhabe am proximalen Ende durch eine Relativverschiebung der beiden Schiebeelemente betätigbar ist, wobei die Handhabe zwei relativ zueinander bewegliche Griffteile aufweist.

Ein derartiges Schiebeschaftinstrument ist aus der DE-A-41 15 937 bekannt.

Ein weiteres Schiebeschaftinstrument dieser Art ist aus dem DE-Firmenprospekt der Karl Storz GmbH & Co., Tuttlingen, "Karl Storz-Endoskope", Abschnitt "Instrumente für Nase und Nasenplastik", Seite N 10 A, bekannt.

Schiebeschaftinstrumente werden in der endoskopischen Chirurgie für operative Eingriffe im menschlichen oder tierischen Körper unterschiedlicher Art eingesetzt, wobei Schiebeschaftinstrumente besonders dafür geeignet sind, große Kräfte zu übertragen, auch unter beengten Platzverhältnissen, etwa zwischen Wirbelknochen.

Das aus dem zuvor genannten DE-Firmenprospekt bekannte Schiebeschaftinstrument weist einen Schiebeschaft auf, der zwei Seite an Seite angeordnete und relativ zueinander verschiebbare Schiebeelemente aufweist. Das eine, unbewegliche Schiebeelement ist dabei fest mit dem unbeweglichen Griffteil verbunden, während das andere Schiebeelement verschiebbar und direkt mit dem beweglichen Griffteil der Handhabe am proximalen Ende verbunden ist. Durch Betätigung der Handhabe wird das bewegliche Schiebeelement relativ zu dem unbeweglichen Schiebeelement verschoben, wodurch sich ein Maulteil am distalen Ende des Schiebeschaftinstrumentes betätigen läßt, um bspw. Gewebe abzutrennen oder zu fassen.

Ein Nachteil des bekannten Schiebeschaftinstrumentes besteht jedoch darin, daß es nur unzureichend oder zumindest nur mit hohem Zeitaufwand in seine Komponenten zerlegbar ist, so daß die Reinigung des Schiebeschaftinstrumentes Schwierigkeiten bereitet. Insbesondere sammeln sich während einer Operation Verunreinigungen zwischen den beiden Schiebeelementen, d.h. genauer zwischen den aufeinanderliegenden Gleitflächen der beiden Schiebeelemente, an. Bei dem bekannten Schiebeschaftinstrument ist der Bereich zwischen den Schiebeelementen nicht zugänglich, so daß nicht gewährleistet ist, daß Verunreinigungen zwischen den Schiebeelementen sicher beseitigt werden können.

Bei längeren Instrumenten, speziell für die abdominale endoskopgestützte Bauchraum-Chirurgie, ist eine einfache Reinigbarkeit des Schiebeschaftinstrumentes jedoch unbedingt notwendig.

Das aus der eingangs genannten DE-A-41 15 937 bekannte Schiebeschaftinstrument weist einen Schiebeschaft auf, der ebenfalls unmittelbar mit der Handhabe verbunden ist. Der Schiebeschaft ist zwar so ausgebildet, daß sich die beiden den Schiebeschaft bildenden Schiebeelemente zur einfacheren Reinigung voneinander trennen lassen, nachdem der Schiebeschaft von der Handhabe abgenommen wurde, jedoch ist dieses bekannte Schiebeschaftinstrument aus folgendem Grund nachteilig.

Bei endoskopgestützten Operationen werden die benötigten Instrumente häufig durch einen Trokar eingesetzt, der eine Öffnung mit kreisförmigem Querschnitt freigibt. Da zur Zugänglichmachung des Operationsfeldes ein Überdruck erzeugt wird, soll ein solches Instrument im Trokar möglichst dicht abschließend und außerdem durch den Trokar sicher geführt werden.

Das zuvor genannte bekannte Schiebeschaftinstrument gewährleistet einen solchen abdichtenden Abschluß in einem Trokar nicht, weil sich der Schiebeschaft bis zur Handhabe erstreckt und somit beim Einsetzen in einen Trokar ein eingestellter Überdruck im Operationsfeld zwischen den beiden Seite an Seite angeordneten Schiebeelementen nach außen entweichen kann. Ein weiterer Nachteil bei einer Verwendung des bekannten Instruments mit einem Trokar besteht darin, daß eines der beiden Schiebeelemente beim Betätigen des Instruments in dem Trokar mit diesem reibend in Kontakt steht.

Ferner ist aus dem DE-GM 94 21 125 eine chirurgische Zange bekannt, die kein Schiebeschaftinstrument im Sinne der vorliegenden Erfindung ist, sondern ein Rohrschaftinstrument, dessen Rohrschaft einen Längsschlitz aufweist, in dem eine Betätigungsstange geführt ist, die in dem Längsschlitz axial beweglich ist und mit dem einen Maulteil am distalen Ende verbunden ist, um dieses zu bewegen. Dieses Rohrschaftinstrument gewährleistet ebenfalls keinen dichten Abschluß in einem Trokar, weil hier ein Überdruck durch den Längsschlitz im Rohrschaft entweichen kann.

Ein herkömmliches Rohrschaftinstrument mit einem geschlossenen Rohrschaft, das jedoch kein Schiebeschaftinstrument ist, ist aus dem DE-GM 17 12 028 bekannt.

Der Erfindung liegt daher die Aufgabe zugrunde, ein Schiebeschaftinstrument der eingangs genannten Art dahingehend weiterzubilden, daß es leicht gereinigt und steril gemacht werden kann, und daß sichergestellt werden kann, daß Verunreinigungen an den Bauteilen, insbesondere zwischen den Schiebeelementen, beseitigt werden können. Ein solches Instrument soll außerdem die Dichtigkeit eines Trokars, durch den es eingeführt wird, gewährleisten und durch den Trokar sicher geführt und gehalten werden. Diese Aufgabe wird hinsichtlich des eingangs genannten Schiebeschaftinstrumentes dadurch gelöst, daß der Schiebeschaft am distalen Ende eines lösbar an der Handhabe befestigten langerstreckten Rohrschaftes angeordnet ist, daß das erste Schiebeelement am distalen Ende des Rohrschaftes ausschwenkbar angebracht ist, daß zum Betätigen des zumindest einen Maulteils der Rohrschaft gemeinsam mit dem ersten Schiebeelement relativ zu dem zweiten Schiebeelement verschiebbar ist, und daß das erste Schiebeelement ausschwenkbar ist, wenn der Rohrschaft von der Handhabe gelöst und ein weiteres Stück bezüglich des zweiten Schiebeelements verschoben wird.

Die erfindungsgemäße Ausgestaltung des Schiebeschaftinstrumentes ermöglicht eine einfache und schnelle Zerlegung des Schiebeschaftinstrumentes und gewährleistet außerdem die erforderliche Dichtigkeit und Führung beim Arbeiten durch einen Trokar. Dadurch, daß zwischen Schiebeelementen und Handhabe ein Rohrschaft vorgesehen ist, paßt das Instrument dicht und sicher in einen üblicherweise zylindrischen Trokar. Dadurch, daß das erste Schiebeelement des Schiebeschaftes am distalen Ende des Rohrschaftes ausschwenkbar angebracht ist, läßt sich zur Reinigung des Schiebeschaftinstrumentes das erste Schiebeelement gegenüber dem zweiten Schiebeelement abklappen, wodurch der Bereich zwischen den beiden Schiebeelementen zur Reinigung leicht zugänglich ist. Zum Reinigen der Schiebeelemente müssen diese demnach nur auseinandergeklappt werden. Da dieses erste Schiebeelement am distalen Ende des Rohrschaftes ausschwenkbar angebracht ist, ist weiterhin erfindungsgemäß vorgesehen, daß zum Betätigen des zumindest einen Maulteils mittels der Handhabe der Rohrschaft verschoben wird, wodurch die Relativverschiebung der beiden Schiebeelemente bewirkt wird, weil das erste Schiebeelement mit dem Rohrschaft verbunden ist. Die erfindungsgemäße verschiebbare Ausgestaltung des Rohrschaftes hat den Vorteil, daß das Schiebeschaftinstrument mit einer Handhabe versehen werden kann, wie sie unter der Bezeichnung "Take Apart"-Griff der Karl Storz GmbH, Tuttlingen, bekannt ist, die sich für medizinische Zangen sehr bewährt hat. Weiterhin ist erfindungsgemäß vorgesehen, daß der Rohrschaft von der Handhabe lösbar ist, wodurch eine weitere Zerlegung des Schiebeschaftinstrumentes ermöglicht wird. Dadurch, daß das erste Schiebeelement nur dann ausschwenkbar ist, wenn der Rohrschaft von der Handhabe gelöst ist, wird erreicht, daß die beiden Schiebeelemente bei einem operativen Gebrauch des Schiebeschaftinstrumentes nicht auseinanderklappen. Insgesamt wird durch die Erfindung ein Schiebeschaftinstrument bereitgestellt, das einfach und schnell soweit zerlegbar ist, daß die einzelnen Komponenten des Schiebeschaftinstrumentes leicht gereinigt werden können und alle Komponenten einer Reinigung soweit zugänglich sind, daß Verunreinigungen sicher entfernt werden können.

Die der Erfindung zugrundeliegende Aufgabe wird somit vollkommen gelöst.

In einer bevorzugten Ausgestaltung ist das erste Griffteil beweglich und der Rohrschaft mit diesem Griffteil verbunden.

Bei dieser Ausgestaltung ist demnach der verschiebbare Rohrschaft mit dem beweglichen Griffteil verbunden, wodurch der Vorteil erzielt wird, daß eine Bewegung des beweglichen Griffteiles direkt in die Bewegung des Rohrschaftes umgesetzt wird und die Zwischenschaltung weiterer mechanischer Betätigungselemente vermieden wird.

In einer weiteren bevorzugten Ausgestaltung ist das zweite Schiebeelement unbeweglich und über ein Stabelement mit dem zweiten unbeweglichen Griffteil verbunden.

Diese Maßnahme hat den Vorteil, daß das Stabelement in dem Rohrschaft angeordnet werden kann, so daß der Rohrschaft das Stabelement gehäuseartig umgibt und keine oder nur geringe Öffnungen gebildet werden, durch die Verunreinigungen in das Schiebeschaftinstrument eindringen können.

In einer weiteren bevorzugten Ausgestaltung sind die beiden Schiebeelemente zum Betätigen des Maulteiles mittels einer Führung relativ zueinander geführt, wobei die Führung bei der Verschiebung des Rohrschaftes um das weitere Stück gelöst wird.

Diese Maßnahme führt zu einem vorteilhaft einfachen mechanischen Aufbau des erfindungsgemäßen Schiebeschaftinstrumentes, bei dem die Führung einerseits bewirkt, daß die beiden Schiebeelemente zur Betätigung des zumindest einen Maulteiles bei einem operativen Eingriff leicht gegeneinander verschoben werden können, ohne sich voneinander zu lösen. Andererseits eröffnet eine Führung eine mechanisch einfache Verbindung, die ein Ausschwenken des ersten Schiebeelementes ermöglicht, wenn der Rohrschaft um das weitere Stück verschoben wird, indem die Führung dann gelöst wird.

Dabei ist es weiterhin bevorzugt, wenn die Führung zumindest eine T-förmige Nut und einen Steg mit dazu komplementärem Querschnitt aufweist, und wobei die Nut zumindest einen erweiterten Abschnitt aufweist, der breiter als die Breite des Stegs ausgebildet ist.

Diese Maßnahme hat den Vorteil, daß die T-förmige Ausgestaltung der Nut und des Stegs eine während eines operativen Einsatzes unlösbare Verbindung der beiden Schiebeelemente gewährleistet, und wobei der erweiterte Abschnitt ein Ausschwenken des ersten Schiebeelementes ermöglicht, wenn der Steg relativ zur Nut soweit verschoben wird, daß er in dem erweiterten Abschnitt der Nut zu liegen kommt.

In einer weiteren bevorzugten Ausgestaltung weist das erste Schiebeelement an seinem proximalen Ende eine Hülse auf, die an dem Rohrschaft lösbar befestigt ist.

Diese Maßnahme hat den Vorteil, daß die Zerlegbarkeit des erfindungsgemäßen Schiebeschaftinstrumentes weiter verbessert ist, weil dann auch der Rohrschaft leicht von dem Schiebeschaft gelöst werden kann.

Dabei ist es bevorzugt, wenn das erste Schiebeelement über ein Gelenk mit der Hülse verbunden ist.

Durch diese Maßnahme wird auf vorteilhafte Weise eine verschwenkbare Befestigung des ersten Schiebeelementes an dem Rohrschaft bewirkt.

In einer weiteren bevorzugten Ausgestaltung ist die Hülse um das zweite Schiebeelement herum angeordnet und im vom Rohrschaft gelösten Zustand auf dem zweiten Schiebeelement verschiebbar.

Diese Maßnahme hat den Vorteil, daß auch Verunreinigungen, die sich zwischen der Innenseite der Hülse und der Außenseite des zweiten Schiebeelementes ansammeln, leicht entfernt werden können, indem die Hülse auf dem zweiten Schiebeelement verschoben wird. Durch diese Maßnahme ist die Reinigbarkeit des erfindungsgemäßen Schiebeschaftinstrumentes weiter verbessert.

In einer weiteren bevorzugten Ausgestaltung ist die Hülse mittels eines bajonettartigen Verschlusses an dem Rohrschaft befestigt.

Diese Maßnahme hat den Vorteil, daß die Hülse durch eine Drehung mit dem Rohrschaft verrastbar ist und von diesem ebenso leicht wieder gelöst werden kann, wodurch eine schnelle Trennung des Rohrschaftes von dem Schiebeschaft erreicht wird.

In einer weiteren bevorzugten Ausgestaltung trägt das erste Schiebeelement an seinem distalen Ende ein erstes Maulteil, und ein zweites Maulteil ist an dem distalen Ende des zweiten Schiebeelementes befestigt, wobei die Maulteile beim Schließen in der Art einer Stanze zusammenwirken.

Bei dieser Ausgestaltung ist das Schiebeschaftinstrument bspw. als Knochen- oder Gewebestanze verwendbar, die den Vorteil einer leichten Zerlegbarkeit und Reinigbarkeit aufweist, die bei herkömmlichen Knochen- und Gewebestanzen bisher nicht erreicht wurden.

In einer alternativen Ausgestaltung ist das erste Schiebeelement an seinem distalen Ende mit einem ersten Maulteil verbunden, das an einem zweiten Maulteil, das mit dem zweiten Schiebeelement verbunden ist, verschwenkbar angelenkt ist, wobei bei einer Verschiebung der Schiebeelemente relativ zueinander die beiden Maulteile in der Art einer Zange zusammenwirken.

Bei dieser erfindungsgemäßen Ausgestaltung kann das Schiebeschaftinstrument als Faß- oder Schneidzange verwendet werden, wobei eine derartige Faß- oder Schneidzange gegenüber herkömmlichen Faß- oder Schneidzangen wiederum den Vorteil aufweist, leichter zerlegbar und reinigbar zu sein.

Dabei ist bevorzugt, wenn das erste Maulteil lösbar mit dem ersten Schiebeelement verbunden ist.

Durch diese Maßnahme wird der Vorteil erzielt, daß das erste Schiebeelement ausgeschwenkt werden kann, ohne daß die beiden miteinander gelenkig verbundenen Maulteile voneinander gelöst werden müssen. Weiterhin hat diese Maßnahme den Vorteil, daß die Maulteile ebenfalls in eine gut reinigbare Stellung gebracht werden können.

In einer weiteren bevorzugten Ausgestaltung wird das erste Maulteil beim Ausschwenken des ersten Schiebeelementes von diesem selbsttätig gelöst.

Durch diese Maßnahme wird der Vorteil erzielt, daß eine Zerlegung des erfindungsgemäßen Schiebeschaftinstrumentes schnell und einfach durchführbar ist, ohne daß dazu eine besondere Geschicklichkeit erfordernde Manipulationen erforderlich sind.

Eine sich derartig selbsttätig lösende Verbindung zwischen dem ersten Maulteil und dem ersten Schiebeelement kann bspw. dadurch erreicht werden, daß an dem ersten Schiebeelement ein Achsstift und an dem ersten Maulteil eine Ausnehmung vorhanden ist, wobei der Achsstift beim Ausschwenken des ersten Schiebeelementes aus der Ausnehmung ausgehoben wird.

In einer weiteren bevorzugten Ausgestaltung können das erste Maulteil von dem zweiten Maulteil und/oder das erste Schiebeelement von der Hülse und/oder die Hülse von dem zweiten Schiebeelement vollständig gelöst werden.

Durch diese Maßnahme wird der Vorteil erzielt, daß alle Oberflächen der Elemente des distalen Endes zur Reinigung und Sterilisation vollständig freigelegt werden können.

Weitere Vorteile ergeben sich aus der nachfolgenden Beschreibung der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach näher beschrieben. Es zeigen:
- Fig. 1: ein Schiebeschaftinstrument gemäß einem ersten Ausführungsbeispiel in einer ersten Betriebsstellung;
- Fig. 2: das distale Ende des Schiebeschaftinstrumentes in Fig. 1 in einer zweiten Betriebsstellung;
- Fig. 3: das distale Ende des Schiebeschaftinstrumentes in Fig. 1 in der Reinigungsstellung;
- Fig. 4: den Rohrschaft des Schiebeschaftinstrumentes in Fig. 1 im von der Handhabe des Schiebeschaftinstrumentes gelösten Zustand;
- Fig. 5: einen Querschnitt entlang der Linie V-V in Fig. 2 durch den Schiebeschaft des Schiebeschaftinstrumentes in Fig. 1;
- Fig. 6: eine Ansicht von unten auf das erste Schiebeelement des Schiebeschaftes des Schiebeschaftinstrumentes in Fig. 1;
- Fig. 7: das distale Ende eines erfindungsgemäßen Schiebeschaftinstrumentes gemäß einem zweiten Ausführungsbeispiel in einer ersten Betriebsstellung;
- Fig. 8: das distale Ende des Schiebeschaftinstrumentes in Fig. 7 in einer zweiten Betriebsstellung; und
- Fig. 9: das distale Ende des Schiebeschaftinstrumentes in Fig. 7 und 8 in der Reinigungsstellung.

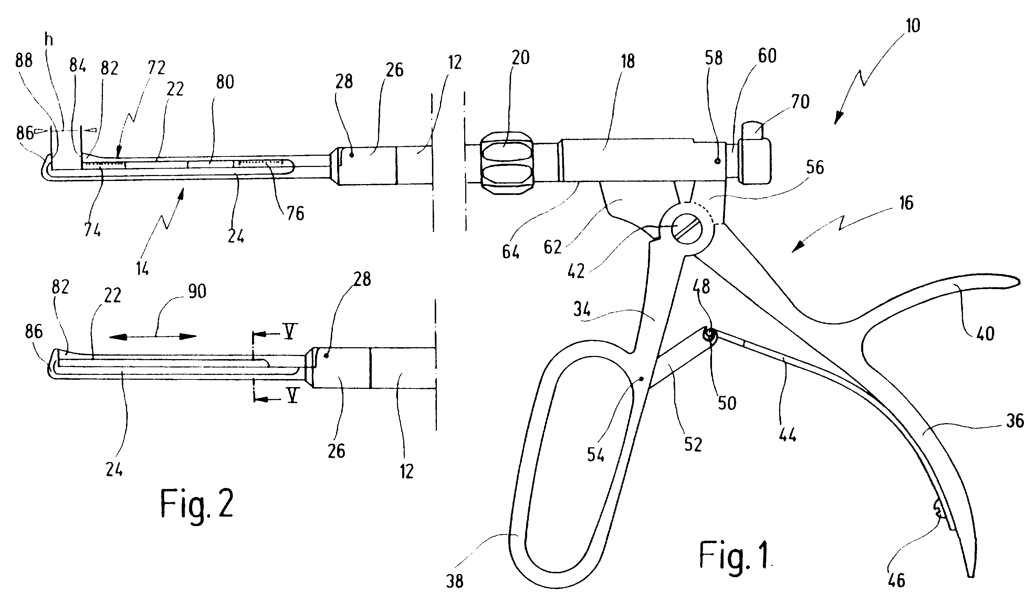

In Fig. 1 ist ein insgesamt mit dem Bezugszeichen 10 versehenes Schiebeschaftinstrument dargestellt. Das Schiebeschaftinstrument 10 wird für endoskopisch-chirurgische Eingriffe im menschlichen oder tierischen Körper verwendet.

Das Schiebeschaftinstrument 10 weist einen Rohrschaft 12, der in Fig. 1 mit einer Unterbrechung dargestellt ist, einen Schiebeschaft 14 am distalen Ende und eine Handhabe 16 am proximalen Ende auf. Der unterbrochen dargestellte Rohrschaft 12 weist insgesamt etwa die drei- bis vierfache Länge des Schiebeschaftes 14 auf.

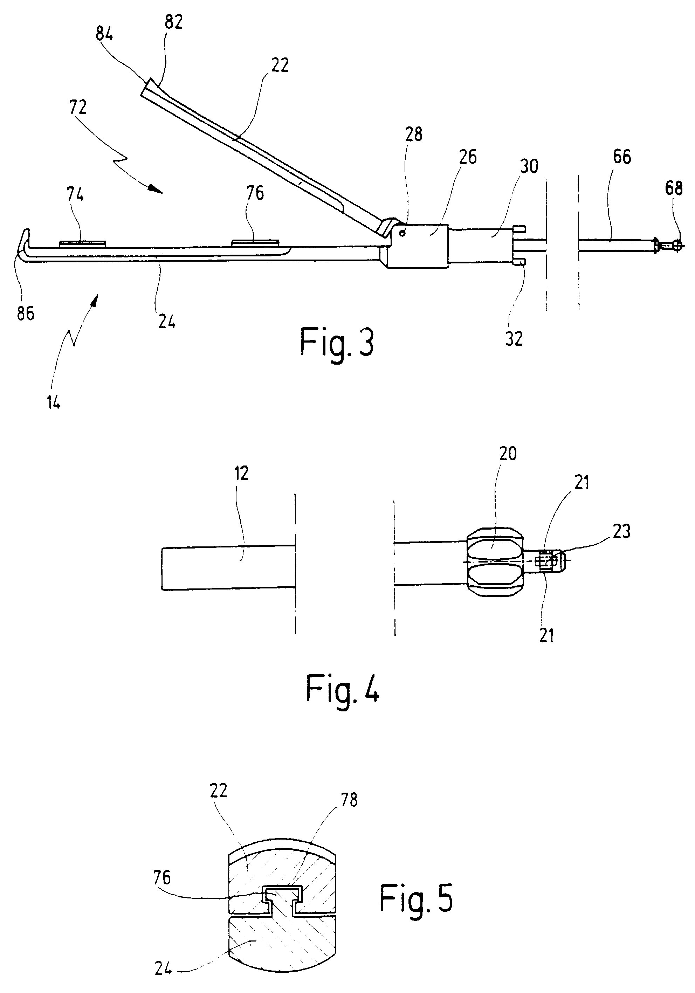

Der Rohrschaft 12 ist mit einem Rohrstück 18 der Handhabe 16 lösbar verbunden. Dazu weist der Rohrschaft 12 eine Überwurfmutter 20 auf, die, wie in Fig. 4 dargestellt ist, am proximalen Ende des Rohrschaftes 12 unverlierbar angeordnet ist. Die Überwurfmutter 20 ist mit einem an dem Rohrstück 18 vorgesehenen nicht dargestellten Gewinde verschraubt (Fig. 1).

Der Schiebeschaft 14 weist ein erstes Schiebeelement 22 und ein zweites Schiebeelement 24 auf, die Seite an Seite angeordnet sind.

Das erste Schiebeelement 22 ist an dem Rohrschaft 12 an dessen distalem Ende ausschwenkbar angebracht, wobei in Fig. 3 das erste Schiebeelement 22 in der ausgeschwenkten Stellung ohne den Rohrschaft 12 dargestellt ist. Das erste Schiebeelement 22 ist an einer Hülse 26 mittels eines Gelenkes 28 befestigt. Die Hülse 26 ist ihrerseits um das zweite Schiebeelement 24 herum angeordnet und relativ zu diesem verschiebbar. Die Hülse 26 weist ferner an ihrem proximalen Ende einen Hülsenfortsatz 30 auf, der an seinem proximalen Ende Vorsprünge 32 trägt. Der Hülsenfortsatz 30 ist in Fig. 1 in das distale Ende des Rohrschafts 12 eingeschoben, wobei in der Innenwand des distalen Endes des Rohrschaftes 12 nicht dargestellte Ausnehmungen vorhanden sind, in die die Vorsprünge 32 eingeführt werden können, und wobei die Hülse 26 mit dem Rohrschaft 12 durch etwa eine Vierteldrehung relativ zu dem Rohrschaft 12 verrastet wird. Die Vorsprünge 32 und die entsprechenden Ausnehmungen bilden somit einen bajonettartigen Verschluß der Hülse 26 an dem Rohrschaft 12.

Die Handhabe 16 weist weiter ein erstes Griffteil 34 und ein zweites Griffteil 36 auf. Das erste Griffteil 34 ist beweglich, während das zweite Griffteil 36 unbeweglich ist.

Das erste Griffteil 34 weist einen Ring 38 auf, durch den ein oder mehrere Finger der Bedienungsperson durchgeführt werden können. Das unbewegliche Griffteil 36 weist ein von diesem nach proximal abstehendes Abstützteil 40 auf. Im Gebrauch des Schiebeschaftinstrumentes 10 bei einem operativen Eingriff liegt das unbewegliche Griffteil 36 zwischen Daumen und Zeigefinger der Hand der Bedienungsperson, wobei das Abstützteil 40 ein Verrutschen der Hand verhindert. Das bewegliche Griffteil 34 ist mit dem unbeweglichen Griffteil 36 über ein Gelenk 42 verbunden. Das Gelenk 42 wird durch eine durch entsprechende Bohrungen in dem beweglichen Griffteil 34 und dem unbeweglichen Griffteil 36 durchgeführte Schraube gebildet, die auf der gegenüberliegenden Seite durch eine Mutter oder dgl. festgelegt ist.

Ferner ist zwischen dem beweglichen Griffteil 34 und dem unbeweglichen Griffteil 36 eine Blattfeder 44 angeordnet, die mittels einer Schraube 46 an dem unbeweglichen Griffteil 36 befestigt ist. Am distalen Ende der Blattfeder 44 ist ein Zapfen 48 angeordnet, der in eine Ausnehmung 50 eines Federhalters 52 eingesetzt ist. Der Federhalter 52 ist seinerseits an dem beweglichen Griffteil 34 über ein Gelenk 54 befestigt. Die Blattfeder 44 bewirkt, daß das bewegliche Griffteil 34 und das unbewegliche Griffteil 36 in ihre in Fig. 1 dargestellte Ruhestellung vorgespannt sind, d.h. daß die Blattfeder 44 die Griffteile 34 und 36 auseinanderdrückt.

Ein Ende 56 des beweglichen Griffteiles 34 ist über ein auch eine geringe seitliche Bewegung zulassendes Gelenk 58 mit dem Rohrstück 18 verbunden. Das Rohrstück 18 sitzt axial verschiebbar in der Art eines Schlittens auf einem unbeweglichen Element 60 der Handhabe 16, an dem ein Ende 62 des unbeweglichen Griffteiles 36 befestigt ist, indem das Ende 62 durch einen auf der Unterseite des Rohrstückes 18 ausgebildeten längsverlaufenden Schlitz 64 hindurchgreift.

Das zweite Schiebeelement 24 ist fest mit einem Stabelement 66 verbunden (vgl. Fig. 3), das an seinem proximalen Ende eine Kugel 68 aufweist, die mit einer entsprechenden nicht dargestellten Ausnehmung in dem unbeweglichen Element 60 in Eingriff steht. Am proximalen Ende des unbeweglichen Elementes 60 ist dazu ein Druckknopf 70 vorgesehen, bei dessen Betätigung durch Herunterdrücken die Kugel 68 in die entsprechende Ausnehmung in dem unbeweglichen Element 60 einführbar ist und bei losgelassenem Druckknopf 70 verriegelt ist.

Das Stabelement 66 weist eine solche Länge auf, daß es den Rohrschaft 12 am proximalen Ende überragt. Das Stabelement 66 verläuft in dem Rohrschaft 12 und einem Abschnitt des unbeweglichen Elementes 60 und ist daher in Fig. 1 nicht sichtbar. Das Stabelement 66 weist im proximalen Bereich eine Abflachung auf, in die zwei am Rohrschaft 12 angebrachte bewegliche Halbschalen 21 eingreifen, die von einem elastischen Ring 23 zusammengehalten werden. Ist das Rohrstück 18 an den Rohrschaft 12 angesetzt, so werden die Halbschalen 21 an die Abflachung gedrückt, so daß eine Drehung und damit Entriegelung der Hülse 26 nicht möglich ist. Damit wird sichergestellt, daß das Zerlegen des Instruments mit dem Abnehmen der Handhabe 16 beginnen muß.

Insgesamt ist das zweite Schiebeelement 24 über das Stabelement 66, das durch den Rohrschaft 12 bis zu der Ausnehmung des unbeweglichen Elementes 60 geführt ist, unbeweglich mit dem unbeweglichen Element 60 und damit mit dem unbeweglichen Griffteil 36 verbunden. Das erste Schiebeelement 22 ist dagegen über die Hülse 26, den Rohrschaft 12 und das Rohrstück 18 mit dem beweglichen Griffteil 34 verbunden, und ist durch eine Verschiebung des Rohrschaftes 12 relativ zu dem zweiten Schiebeelement 24 verschiebbar.

Das erste Schiebeelement 22 und das zweite Schiebeelement 24 sind im Gebrauchszustand des Schiebeschaftinstrumentes 10 über eine Führung 72 miteinander verbunden. Das zweite Schiebeelement 24 weist dazu zwei axial voneinander beabstandete längsverlaufende Stege 74 und 76 auf, die in eine in dem ersten Schiebeelement 22 ausgebildete Nut 78 eingreifen. Die Stege 74 und 76 weisen einen T-förmigen Querschnitt auf, wobei die Nut 78 einen dazu komplementären, ebenfalls T-förmigen Querschnitt aufweist (vgl. Fig. 5). Die Nut 78 weist einen erweiterten Abschnitt 80 auf, der eine Öffnung definiert, die breiter als die Gesamtbreite des Stegs 76 ist. Der Steg 76 läßt sich somit durch den erweiterten Abschnitt 80 in die Nut 78 einsetzen, wie später noch näher erläutert wird. Ferner ist die Nut 78 am distalen Ende des ersten Schiebeelementes 22 offen.

Ferner weist das erste Schiebeelement 22 an seinem distalen Ende ein erstes Maulteil 82 auf, an dessen distalem Ende eine Schneidkante 84 ausgebildet ist. Das zweite Schiebeelement 24 weist ein zweites Maulteil 86 auf, das an seinem proximalen Ende eine Schneidkante 88 aufweist.

Im folgenden wird nun die Funktionsweise des Schiebeschaftinstrumentes 10 beschrieben.

In Fig. 1 ist das Schiebeschaftinstrument 10 in einer ersten Betriebsstellung, d.h. der Ruhestellung, dargestellt. In diesem Zustand liegen das erste Schiebeelement 22 und das zweite Schiebeelement 24 Seite an Seite aufeinander, wobei die Stege 74 und 76 in die Nut 78 eingreifen. In Fig. 1 ist ferner mit h die maximale Offenstellung der Maulteile 82 und 86 im operativen Einsatz angedeutet, die gleichzeitig dem Arbeitshub des Schiebeschaftes 14, d.h. genauer des ersten Schiebeelementes 22 entspricht.

Wird nun das bewegliche Griffteil 34 in Richtung zu dem unbeweglichen Griffteil 36 bewegt, verschiebt das Ende 56 des beweglichen Griffteiles 34, das an dem beweglichen Rohrstück 18 angreift, den Rohrschaft 12 in Richtung distales Ende. Durch die Verschiebung des Rohrschaftes 12 wird auch das erste Schiebeelement 22, das über die Hülse 26 mit dem Rohrschaft 12 verbunden ist, zum distalen Ende hin verschoben, wodurch sich das Maulteil 82 zu dem Maulteil 86 verschiebt. Die Schneidkanten 84 und 88 wirken beim Schließen der Maulteile 82 und 86 in der Art einer Stanze zusammen, so daß das Schiebeschaftinstrument 10 zum Stanzen von Knochen oder Gewebe verwendet werden kann. Hierbei kann eine sehr große Kraft aufgebracht werden, da das relativ dünne Stabelement 66 dabei nur auf Zug belastet wird. In Fig. 2 ist das distale Ende des Schiebeschaftinstrumentes 10 in einer Betriebsstellung dargestellt, in der die Maulteile 82 und 86 vollständig geschlossen sind und der Rohrschaft 12 relativ zu dem zweiten Schiebeelement 24 maximal zum distalen Ende hin verschoben ist.

Wird die auf das bewegliche Griffteil 34 ausgeübte Handkraft wieder verringert, bewirkt die Blattfeder 44, daß sich das bewegliche Griffteil 34 wieder in seine Ausgangsstellung zurückbewegt, wodurch der Rohrschaft 12 wieder zum proximalen Ende des Schiebeschaftinstrumentes 10 hin verschoben bzw. gezogen wird. Dabei öffnen sich die Maulteile 82 und 86 wieder.

Durch die Bewegung des beweglichen Griffteiles 34 auf das unbewegliche Griffteil 36 zu und von diesem weg wird das erste Schiebeelement 22 somit gemäß einem Doppelpfeil 90 relativ zu dem zweiten Schiebeelement 24 zur Betätigung der Maulteile 84 und 86 verschoben. Während dieser Verschiebung stehen die Stege 74 und 76 des zweiten Schiebeelementes 24 über den gesamten Arbeitshub h mit der Nut 78 des ersten Schiebeelementes 22 derart in Eingriff, daß die Stege 74 und 76 nicht in den erweiterten Abschnitt 80 der Nut 78 gelangen oder aus dem distalen Ende der Nut 78 austreten, wodurch das erste Schiebeelement 22 nicht unerwünscht ausschwenken kann.

Im folgenden wird nun beschrieben, wie das Schiebeschaftinstrument 10 zerlegt werden kann.

Zunächst wird die Überwurfmutter 20 vollkommen lose gedreht. Danach wird der Druckknopf 70 heruntergedrückt, wodurch sich die Kugel 68 am proximalen Ende des Stabelementes 66 von dem unbeweglichen Element 60 abziehen läßt. Die Baugruppe bestehend aus dem Schiebeschaft 14, dem Rohrschaft 12 und dem Stabelement 66 läßt sich dann von der Handhabe 16 zusammen abziehen. Die Halbschalen 21 am Rohrschaft 12 sind dann freigegeben.

Indem nun der Rohrschaft 12 relativ zu dem zweiten Schiebeelement 24 um ein weiteres Stück zum proximalen Ende hin verschoben wird, und zwar um die Länge des Stegs 74 bzw. des Stegs 76, tritt der Steg 74 aus der Nut 78 aus deren distalseitiger Öffnung aus, während der Steg 76 in dem erweiterten Abschnitt 80 der Nut 78 zu liegen kommt. Nun läßt sich das erste Schiebeelement 22 über das Gelenk 28 an der Hülse 26 von dem zweiten Schiebeelement 24 abklappen. Somit kann der Schiebeschaft 14 besonders leicht im Bereich zwischen dem ersten Schiebeelement 22 und dem zweiten Schiebeelement 24 gereinigt werden.

Der Schiebeschaft 14 läßt sich weiter von dem Rohrschaft 12 abnehmen, indem die Hülse 26 um eine Vierteldrehung gegenüber dem Rohrschaft 12 gedreht wird, wodurch der durch die Vorsprünge 32 gebildete bajonettartige Verschluß gelöst wird. In Fig. 3 ist der Rohrschaft 12 bereits von dem Schiebeschaft 14 abgenommen, während das Stabelement 66 an dem zweiten Schiebeelement 24 fest verbunden bleibt. Die Hülse 26 läßt sich nun noch auf dem zweiten Schiebeelement 24 axial verschieben, so daß eine Reinigung der Innenseite der Hülse 26 und des Bereiches des zweiten Schiebeelementes 24, auf dem die Hülse 26 während des Betriebes axial verschiebbar ist, leicht durchführbar ist. Somit können alle schwer zugänglichen Bereiche des Schiebeschaftinstrumentes 10 so zerlegt oder freigelegt werden, daß gewährleistet ist, daß auch Verunreinigungen in diesen Bereichen sicher entfernt werden können und das Schiebeschaftinstrument 10 steril gemacht werden kann.

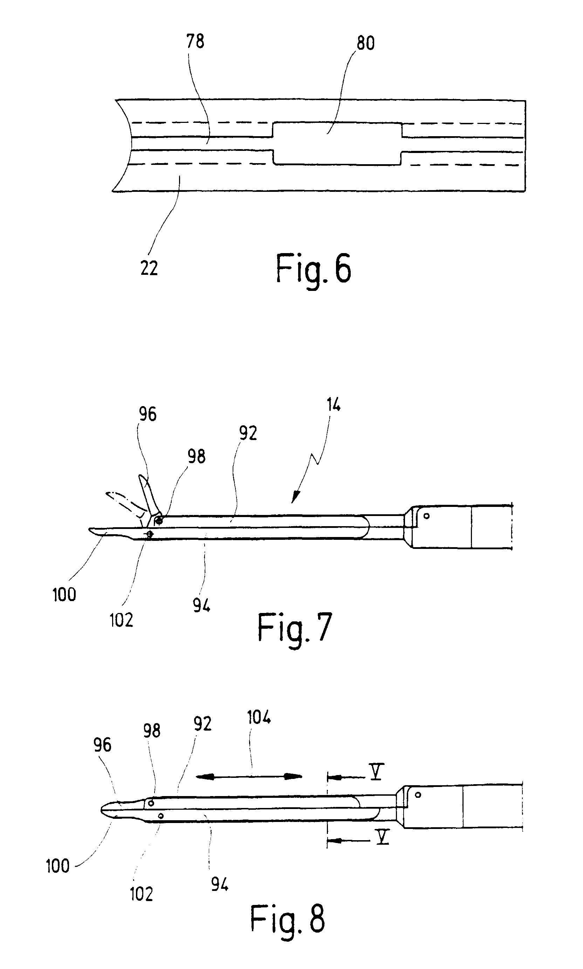

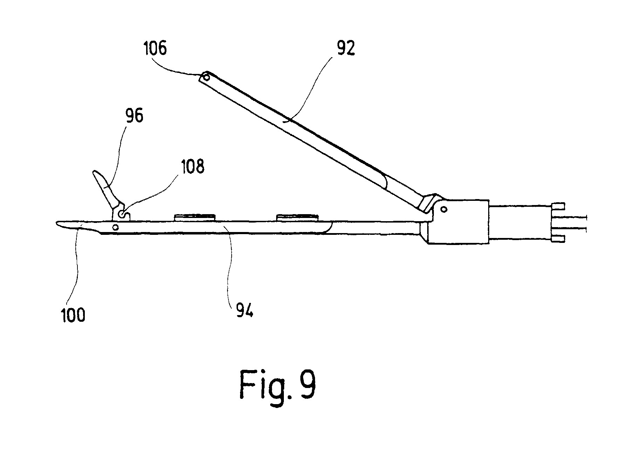

In Fig. 7 bis 9 ist ein weiteres Ausführungsbeispiel dargestellt, das sich von dem in Fig. 1 bis 6 dargestellten Ausführungsbeispiel lediglich hinsichtlich der Maulteile am distalen Ende des Schiebeschaftes 14 unterscheidet. Bei einer derartigen Ausgestaltung der Maulteile kann das Schiebeschaftinstrument 10 als Schneid- oder Faßzange verwendet werden.

So ist am distalen Ende eines ersten Schiebeelementes 92, das wiederum gegenüber einem zweiten Schiebeelement 94 verschiebbar ist, ein erstes Maulteil 96 angeordnet, das über ein Gelenk 98 mit dem ersten Schiebeelement 92 verbunden ist. Weiterhin ist das erste Maulteil 96 an einem zweiten Maulteil 100 über ein Gelenk 102 angelenkt.

Durch eine Verschiebung des ersten Schiebeelementes 92 relativ zu dem zweiten Schiebeelement 94 gemäß einem Doppelpfeil 104 wird das erste Maulteil 96 gegen das zweite Maulteil 100 geschlossen, wie in Fig. 8 dargestellt ist, oder, wie in Fig. 7 dargestellt ist, geöffnet. Die Schließbewegung des ersten Maulteiles 96 wird dadurch erreicht, daß durch die axiale Verschiebung des ersten Schiebeelementes 92 das Gelenk 98 gegenüber dem Gelenk 102 zum distalen Ende hin verschoben wird, wodurch sich das erste Maulteil 96 um das Gelenk 102 verschwenkt. Auch in dieser Ausführung kann die Schließbewegung mit sehr großer Kraft erfolgen, da das relativ dünne Stabelement 66 wiederum nur auf Zug belastet wird.

Beim Verschieben des ersten Schiebeelementes 92 um ein weiteres Stück, was wie bei dem ersten Ausführungsbeispiel erst nach dem Lösen der Handhabe 16 möglich ist, wird die Verbindung des ersten Schiebeelementes 92 mit dem ersten Maulteil 96 im Bereich des Gelenkes 98 gelöst. Dazu ist das Gelenk 98 dadurch gebildet, daß an dem ersten Schiebeelement 92 ein Achsstift 106 befestigt ist, der in eine Ausnehmung 108 an dem ersten Maulteil 96 eingreift und beim Verschieben um ein weiteres Stück selbsttätig aus der Ausnehmung 108 austritt.

Das erste Schiebeelement 92 ist wiederum wie bei dem ersten Ausführungsbeispiel ausschwenkbar ausgebildet. Zum Ausschwenken des ersten Schiebeelementes 92 wird der Rohrschaft gegenüber dem zweiten Schiebeelement 94 noch weiter zum proximalen Ende hin verschoben, wobei die Führung gelöst wird.

## Patentansprüche

1. Medizinisches Schiebeschaftinstrument, mit einem Schiebeschaft (14), der zwei Seite an Seite angeordnete und relativ zueinander axial verschiebbare voneinander trennbare Schiebeelemente (22, 24; 92, 94) aufweist, mit zumindest einem Maulteil (82, 86; 96, 100) am distalen Ende des Schiebeschafts (14), das mittels einer Handhabe (16) am proximalen Ende durch eine Relativverschiebung der beiden Schiebeelemente (22, 24; 92, 94) betätigbar ist, wobei die Handhabe (16) zwei relativ zueinander bewegliche Griffteile (34, 36) aufweist, dadurch gekennzeichnet, daß der Schiebeschaft (14) am distalen Ende eines lösbar an der Handhabe (16) befestigten langerstreckten Rohrschaftes (12) angeordnet ist, daß das erste Schiebeelement (22; 92) am distalen Ende des Rohrschaftes (12) ausschwenkbar angebracht ist, daß zum Betätigen des zumindest einen Maulteils (82, 86; 96, 100) der Rohrschaft (12) gemeinsam mit dem ersten Schiebeelement (22; 92) relativ zu dem zweiten Schiebeelement (24; 94) verschiebbar ist, und daß das erste Schiebeelement (22; 92) ausschwenkbar ist, wenn der Rohrschaft (12) von der Handhabe (16) gelöst und ein weiteres Stück bezüglich des zweiten Schiebeelements (24; 94) verschoben wird.

2. Schiebeschaftinstrument nach Anspruch 1, dadurch gekennzeichnet, daß das erste Griffteil (34) beweglich ist und der Rohrschaft (12) mit diesem Griffteil (34) verbunden ist.

3. Schiebeschaftinstrument nach Anspruch 1 oder 2, dadurch gekennzeichnet, daß das zweite Schiebeelement (24; 94) unbeweglich ist und über ein Stabelement (66) mit dem zweiten, unbeweglichen Griffteil (36) verbunden ist.

4. Schiebeschaftinstrument nach einem der Ansprüche 1 bis 3, dadurch gekennzeichnet, daß die beiden Schiebeelemente (22, 24; 92, 94) zum Betätigen des Maulteils (82, 86; 96, 100) mittels einer Führung (72) relativ zueinander geführt sind, wobei die Führung (72) bei der Verschiebung des Rohrschaftes (12) um das weitere Stück gelöst wird.

5. Schiebeschaftinstrument nach Anspruch 4, dadurch gekennzeichnet, daß die Führung (72) zumindest eine Nut (78) und zumindest einen Steg (74, 76) aufweist, wobei die Nut (78) einen T-förmigen und der Steg (74, 76) einen dazu komplementären Querschnitt aufweist, und wobei die Nut (78) einen erweiterten Abschnitt (80) aufweist, der breiter als die Breite des Stegs (74, 76) ausgebildet ist.

6. Schiebeschaftinstrument nach einem der Ansprüche 1 bis 5, dadurch gekennzeichnet, daß das erste Schiebeelement (22; 92) an seinem proximalen Ende eine Hülse (26) aufweist, die an dem Rohrschaft (12) lösbar befestigt ist.

7. Schiebeschaftinstrument nach Anspruch 6, dadurch gekennzeichnet, daß das erste Schiebeelement (22; 92) von der Hülse (26) vollständig lösbar ist.

8. Schiebeschaftinstrument nach Anspruch 6 oder 7, dadurch gekennzeichnet, daß das erste Schiebeelement (22; 92) über ein Gelenk (28) mit der Hülse (26) verbunden ist.

9. Schiebeschaftinstrument nach einem der Ansprüche 6 bis 8, dadurch gekennzeichnet, daß die Hülse (26) um das zweite Schiebeelement (24; 94) herum angeordnet und im vom Rohrschaft (12) gelösten Zustand auf dem zweiten Schiebeelement (24; 94) verschiebbar ist.

10. Schiebeschaftinstrument nach Anspruch 9, dadurch gekennzeichnet, daß die Hülse (26) von dem zweiten Schiebeelement (24; 94) vollständig lösbar ist.

11. Schiebeschaftinstrument nach einem der Ansprüche 6 bis 10, dadurch gekennzeichnet, daß die Hülse (26) mittels eines bajonettartigen Verschlusses an dem Rohrschaft (12) befestigt ist.

12. Schiebeschaftinstrument nach einem der Ansprüche 1 bis 11, dadurch gekennzeichnet, daß das erste Schiebeelement (22) an seinem distalen Ende das Maulteil (82) trägt, und daß ein zweites Maulteil (86) am distalen Ende des zweiten Schiebeelementes (24) befestigt ist, wobei die Maulteile (82, 86) in der Art einer Stanze zusammenwirken.

13. Schiebeschaftinstrument nach einem der Ansprüche 1 bis 11, dadurch gekennzeichnet, daß das erste Schiebeelement (92) an seinem distalen Ende das Maulteil (96) trägt, das an einem am distalen Ende des zweiten Schiebeelementes (94) befestigten Maulteil (100) verschwenkbar angelenkt ist, wobei bei einer Verschiebung der Schiebeelemente (92, 94) relativ zueinander die beiden Maulteile (96, 100) in der Art einer Zange zusammenwirken.

14. Schiebeschaftinstrument nach Anspruch 13, dadurch gekennzeichnet, daß das erste Maulteil (96) lösbar mit dem ersten Schiebeelement (92) verbunden ist.

15. Schiebeschaftinstrument nach Anspruch 13, dadurch gekennzeichnet, daß das erste Maulteil (96) vollständig von dem ersten Schiebeelement (22; 92) lösbar ist.

16. Schiebeschaftinstrument nach einen der Ansprüche 13 bis 15, dadurch gekennzeichnet, daß das erste Maulteil (96) beim Ausschwenken des ersten Schiebeelementes (92) von diesem selbsttätig gelöst wird.

## Claims

1. A sliding-shaft medical instrument, comprising a sliding shaft (14) that has two slide elements (22, 24; 92, 94), arranged side by side and displaceable axially relative to one another and separable from one another, further comprising at least one jaw part (82, 86; 96, 100) at the distal end of the sliding shaft (14), which is actuable, by way of a handle (16) at the proximal end, via a relative displacement of the two slide elements (22, 24; 92, 94), the handle (16) having two handle elements (34, 36) which are movable relative to one another, characterized in that the sliding shaft (14) is arranged at the distal end of an elongated tubular shaft (12) which is attached detachably to the handle (16), that the first slide element (22; 92) is mounted at the distal end of the tubular shaft (12) in swing-aside fashion, that, in order to actuate the at least one jaw part (82, 86; 96, 100), the tubular shaft (12) is displaceable, together with the first slide element (22; 92), relative to the second slide element (24; 94), and that the first slide element (22; 92) can be swung aside when the tubular shaft (12) is detached from the handle (16) and is displaced a further distance with respect to the second slide element (24; 94).

2. The sliding-shaft instrument of claim 1, characterized in that the first handle element (34) is movable and the tubular shaft (12) is joined to that handle element (34).

3. The sliding-shaft instrument of claim 1 or 2, characterized in that the second slide element (24; 94) is immovable and is joined via a rod element (66) to the second, immovable handle element (36).

4. The sliding-shaft instrument of claims 1 through 3, characterized in that for actuation of the jaw part (82, 86; 96, 100), the two slide elements (22, 24; 92, 94) are guided relative to one another by way of a guide (72), the guide (72) being detached upon displacement of the tubular shaft (12) over the further distance.

5. The sliding-shaft instrument of claim 4, characterized in that the guide (72) has at least one groove (78) and at least one bar (74, 76), the groove (78) having a T-shaped cross section and the bar (74, 76) a cross section complementary thereto, the groove (78) having an enlarged portion (80) which is wider than the width of the bar (74, 76).

6. The sliding-shaft instrument of Claims 1 through 5, characterized in that the first slide element (22; 92) has at its proximal end a sleeve (26) that is detachably attached to the tubular shaft (12).

7. The sliding-shaft instrument of claim 6, characterized in that the first slide element (22; 92) is completely detachable from the sleeve (26).

8. The sliding-shaft instrument of claim 6 or 7, characterized in that the first slide element (22; 92) is joined to the sleeve (26) via a pivot joint (28).

9. The sliding-shaft instrument of claims 6 through 8, characterized in that the sleeve (26) is arranged around the second slide element (24; 94) and, in the state detached from the tubular shaft (12), is displaceable on the second slide element (24; 94).

10. The sliding-shaft instrument of claim 9, characterized in that the sleeve (26) is completely detachable from the second slide element (24; 94).

11. The sliding-shaft instrument of claims 6 through 10, characterized in that the sleeve (26) is attached to the tubular shaft (12) by way of a bayonet-like connector.

12. The sliding-shaft instrument of claims 1 through 11, characterized in that the first slide element (22) carries at its distal end the jaw part (82), and a second jaw part (86) is attached at the distal end of the second slide element (24), the jaw parts (82, 86) coacting in the manner of a punch when closing.

13. The sliding-shaft instrument of claims 1 through 11, characterized in that the first slide element (92) carries at its distal end the jaw part (96), which is pivotably articulated on a jaw part (100) attached at the distal end of the second slide element (94), displacement of the slide elements (92, 94) relative to one another causing the two jaw parts (96, 100) to coact in the manner of a forceps.

14. The sliding-shaft instrument of claim 13, characterized in that the first jaw part (96) is detachably joined to the first slide element (92).

15. The sliding-shaft instrument of claim 13, characterized in that the first jaw part (96) is completely detachable from the first slide element (22; 92).

16. The sliding-shaft instrument of claims 13 through 15, characterized in that the first jaw part (96) is automatically detached from the first slide element (92) as the latter swings aside.

## Revendications

1. Instrument médical à tige coulissante, comportant une tige coulissante (14) qui présente des éléments coulissants (22, 24 ; 92, 94) disposés côte à côte et qui peuvent être séparés l'un de l'autre et coulisser axialement l'un par rapport à l'autre, comportant, à l'extrémité distale de la tige coulissante (14), au moins une partie formant mâchoire (82, 86 ; 96, 100) qui peut être actionnée au moyen d'une poignée (16), à l'extrémité proximale, par un coulissement relatif des deux éléments coulissants (22, 24 ; 92, 94), dans lequel la poignée (16) comporte deux branches (34, 36) déplaçables l'une par rapport à l'autre, caractérisé en ce que la tige coulissante (14) est disposée à l'extrémité distale d'une tige tubulaire (12) allongée, fixée de manière non permanente à la poignée (16), en ce que le premier élément (22 ; 92) est monté de manière à pouvoir pivoter vers l'extérieur à l'extrémité distale de la tige tubulaire (12), en ce que pour actionner la ou les partie(s) formant mâchoires (82, 86 ; 96, 100), la tige tubulaire (12) peut coulisser avec le premier élément coulissant (22 ; 92), par rapport au deuxième élément coulissant (24 ; 94), et en ce que le premier élément coulissant (22 ; 92) peut pivoter vers l'extérieur lorsque la tige tubulaire (12) est séparée de la poignée (16) et est déplacée encore sur une distance complémentaire par rapport au deuxième élément coulissant (24 ; 94).

2. Instrument à tige coulissante selon la revendication 1, caractérisé en ce que la première branche de poignée (34) est déplaçable et la tige tubulaire (12) est reliée à cette branche (34).

3. Instrument à tige coulissante selon la revendication 1 ou 2, caractérisé en ce que le deuxième élément coulissant (24 ; 94) n'est pas déplaçable et est relié à la deuxième branche de poignée (36) non déplaçable, par un élément en barre (66).

4. Instrument à tige coulissante selon l'une des revendications 1 à 3, caractérisé en ce que les deux éléments coulissants (22, 24 ; 92, 84), destinés à actionner la partie formant mâchoire (82, 86 ; 96, 100), sont guidés l'un par rapport à l'autre au moyen d'un dispositif de guidage (72), le dispositif de guidage (72) étant séparé de la distance complémentaire lors du coulissement de la tige tubulaire (12).

5. Instrument à tige coulissante selon la revendication 4, caractérisé en ce que le dispositif de guidage (72) présente au moins une rainure (78) et au moins une nervure (74, 76), la rainure (78) présentant une section en T et la nervure (74, 76) une section complémentaire à celle-ci, et la rainure (78) présentant un segment (80) élargi qui est plus large que ne l'est la nervure (74, 76).

6. Instrument à tige coulissante selon l'une des revendications 1 à 5, caractérisé en ce que le premier élément coulissant (22 ; 92) présente, à son extrémité proximale, un manchon (26) qui est fixé de manière non permanente à la tige tubulaire (12).

7. Instrument à tige coulissante selon la revendication 6, caractérisé en ce que le premier élément coulissant (22 ; 92) est totalement détachable du manchon (26).

8. Instrument à tige coulissante selon la revendication 6 ou 7, caractérisé en ce que le premier élément coulissant (22 ; 92) est relié au manchon (26) par une articulation (28).

9. Instrument à tige coulissante selon l'une des revendications 6 à 8, caractérisé en ce que le manchon (26) est disposé autour du deuxième élément coulissant (24 ; 94) et peut se déplacer sur le deuxième élément coulissant (24 ; 94), à l'état détaché de la tige tubulaire (12).

10. Instrument à tige coulissante selon la revendication 9, caractérisé en ce que le manchon (26) est totalement détachable du deuxième élément coulissant (24 ; 94).

11. Instrument à tige coulissante selon l'une des revendications 6 à 10, caractérisé en ce que le manchon (26) est fixé sur la tige tubulaire (12) au moyen d'une fermeture du type à baïonnette.

12. Instrument à tige coulissante selon l'une des revendications 1 à 11, caractérisé en ce que le premier élément coulissant (22) porte la partie formant mâchoire (82) à son extrémité distale, et en ce qu'une deuxième partie formant mâchoire (86) est fixée à l'extrémité distale du deuxième élément coulissant (24), les parties formant mâchoires (82, 86) coopérant à la manière d'un emporte-pièce.

13. Instrument à tige coulissante selon l'une des revendications 1 à 11, caractérisé en ce que le premier élément coulissant (92) porte, à son extrémité distale, la partie formant mâchoire (96) qui est articulée, de manière à pouvoir pivoter, sur une partie formant mâchoire (100) fixée à l'extrémité distale du deuxième élément coulissant (94), les deux parties formant mâchoires (96, 100) coopérant à la manière d'une pince lors d'un coulissement des éléments coulissants (92, 94) l'un par rapport à l'autre.

14. Instrument à tige coulissante selon la revendication 13, caractérisé en ce que la première partie formant mâchoire (96) est reliée de manière non permanente au premier élément coulissant (92).

15. Instrument à tige coulissante selon la revendication 13, caractérisé en ce que la première partie formant mâchoire (96) est totalement détachable du premier élément coulissant (22 ; 92).

16. Instrument à tige coulissante selon l'une des revendications 13 à 15, caractérisé en ce que la première partie formant mâchoire (96) est automatiquement détachée du premier élément coulissant (92), lorsque celui-ci pivote vers l'extérieur.
